(19) Europäisches Patentamt
European Patent Office
Office européen des brevets

(11) **EP 4 394 783 A1**

(12) **EUROPEAN PATENT APPLICATION**

(43) Date of publication:
**03.07.2024   Bulletin 2024/27**

(21) Application number: **23219067.8**

(22) Date of filing: **21.12.2023**

(51) International Patent Classification (IPC):
***G16C 60/00*** *(2019.01)*

(52) Cooperative Patent Classification (CPC):
**G16C 60/00;** G06N 3/0455; G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022   IN 202221074995**

(71) Applicant: **Tata Consultancy Services Limited Maharashtra (IN)**

(72) Inventors:
• **PILLAI, Prajith**
  **560066 Bangalore, Karnataka (IN)**
• **PAL, Parama**
  **560066 Bangalore, Karnataka (IN)**

(74) Representative: **Goddar, Heinz J.**
  **Boehmert & Boehmert**
  **Anwaltspartnerschaft mbB**
  **Pettenkoferstrasse 22**
  **80336 München (DE)**

(54) **METHOD AND SYSTEM FOR INVERSELY PREDICTING NANOFEATURES OF PLASMONIC METASURFACE USING REMODELED VARIATIONAL AUTOENCODER**

(57)   Currently approaches for inversely obtaining plasmonic meta unit dimensions for given structural color require a considerably large dataset for inverse prediction. Further, they also suffer from low accuracy in regions corresponding to x and y coordinates proximal to the 45-degree axis in a CIE 1931 colour space i.e., close to the white point. Present disclosure provides method and system for inversely predicting nanofeatures of plasmonic metasurface using remodeled variational autoencoder. The system uses a remodeled variational autoencoder (VAE) network that inversely predicts plasmonic metasurface nanofeatures while providing diversity as well as prediction accuracy. The remodeled VAE network has a probabilistic encoder that maps chromaticity coordinates to a latent space and a probabilistic decoder that maps this latent space back to the coordinates. Further, a multi-layer perceptron layer is added to the latent space that considers the reconstruction as well as dimensional prediction, thereby making training of the network versatile.

FIG. 5

**Description**

CROSS-REFERENCE TO RELATED APPLICATIONS AND PRIORITY

The present application claims priority to Indian application no. 202221074995, filed on December 23, 2022.

TECHNICAL FIELD

The disclosure herein generally relates to plasmonic metasurface nanofeature prediction, and, more particularly, to a method and a system for inversely predicting nanofeatures of a plasmonic metasurface using a remodeled variational autoencoder.

BACKGROUND

Plasmonic metamaterials (or plasmonic metasurfaces) are artificially engineered, two-dimensional nanocomposites that have an ability to demonstrate structural color effects. In particular, the plasmonic metamaterials has the ability to provide selective coloration, which makes them an ideal material for applications such as intelligent displays for augmented reality (AR)/virtual reality (VR) devices and high-volume consumer electronics. Further, the use of plasmonic effects of the plasmonic metamaterials to produce desired colors is appeared to be a robust and a cost-effective scheme when compared to the traditional ways of mixing different colorants together.

Basically, the plasmonic metasurfaces comprise of periodic, sub-wavelength unit cells or 'metaunits' that interact with incoming radiation in unconventional ways to provide colored metasurface films. So, to achieve selective coloration of the plasmonic metasurface, the unit cell dimensions i.e., the geometries need to be designed appropriately. However, the process of designing the unit cell dimensions of the plasmonic metamaterial for the desired coloration is a lengthy and iterative one.

Nowadays, data-driven inverse methods have been widely used for designing plasmonic metamaterial 'by specification', i.e., by inversely mapping the desired functionality i.e., the electromagnetic responses for designing the unit cell dimensions of the plasmonic metamaterial. However, the inverse models for designing plasmonic metamaterials remain a challenge due to the large simulation times required for generating the datasets.

Tandem autoencoders also are in use for inversely obtaining plasmonic meta unit dimensions for given structural color(s). However, the disadvantage of the tandem autoencoder is that they require a considerably large dataset for inverse prediction. In addition, the colour space prediction suffers from low accuracy in regions corresponding to x and y coordinates proximal to the 45-degree axis in a CIE 1931 colour space i.e., close to an achromatic 'white' point.

SUMMARY

Embodiments of the present disclosure present technological improvements as solutions to one or more of the above-mentioned technical problems recognized by the inventors in conventional systems. For example, in one aspect, there is provided a processor implemented method for inversely predicting nanofeatures of a plasmonic metasurface using a remodeled variational autoencoder. The method comprises receiving, by a nanofeature prediction system (NPS) via one or more hardware processors, a spectral response data associated with a plasmonic metasurface, the plasmonic metasurface comprising a plurality of metaunits; converting, by the NPS via the one or more hardware processors, the spectral response data into a coordinate space, the coordinate space comprising a plurality of two-dimensional (2D) chromaticity coordinates, wherein each 2D chromaticity coordinate is associated with a dimension information of a metaunit of the plurality of metaunits; preprocessing, by the NPS via the one or more hardware processors, the plurality of 2D chromaticity coordinates using a Euclidean filter to obtain a plurality of preprocessed chromaticity coordinates; and training, by the NPS via the one or more hardware processors, a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss.

In another aspect, there is provided a nanofeature prediction system for inversely predicting nanofeatures of a plasmonic metasurface using a remodeled variational autoencoder. The system comprises a memory storing instructions; one or more communication interfaces; and one or more hardware processors coupled to the memory via the one or more communication interfaces, wherein the one or more hardware processors are configured by the instructions to: receive a spectral response data associated with a plasmonic metasurface, the plasmonic metasurface comprising a plurality of metaunits; convert the spectral response data into a coordinate space, the coordinate space comprising a plurality of two-dimensional (2D) chromaticity coordinates, wherein each 2D chromaticity coordinate is associated with a dimension information of a metaunit of the plurality of metaunits; preprocess the plurality of 2D chromaticity coordinates using a Euclidean filter to obtain a plurality of preprocessed chromaticity coordinates; and train a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss.

In yet another aspect, there are provided one or more non-transitory machine-readable information storage mediums comprising one or more instructions which when executed by one or more hardware processors cause a method for inversely predicting nanofeatures of a plasmonic metasurface using a remodeled variational autoencoder. The method comprises receiving, by a nanofeature prediction system (NPS), a spectral response data associated with a plasmonic metasurface, the plasmonic metasurface comprising a plurality of metaunits; converting, by the NPS, the spectral response data into a coordinate space, the coordinate space comprising a plurality of two-dimensional (2D) chromaticity coordinates, wherein each 2D chromaticity coordinate is associated with a dimension information of a metaunit of the plurality of metaunits; preprocessing, by the NPS, the plurality of 2D chromaticity coordinates using a Euclidean filter to obtain a plurality of preprocessed chromaticity coordinates; and training, by the NPS, a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a schematic representation of a polydimethylsiloxane metaunit of a plasmonic metasurface, related to at least some example embodiments of the present disclosure.

FIG. 2 is an example representation of an environment, related to at least some example embodiments of the present disclosure.

FIG. 3 illustrates an exemplary block diagram of a system for inversely predicting nanofeatures of a plasmonic metasurface using a remodeled variational autoencoder, in accordance with an embodiment of the present disclosure.

FIG. 4 illustrates an exemplary flow diagram of a method for inversely predicting nanofeatures of a plasmonic metasurface using the remodeled variational autoencoder, in accordance with an embodiment of the present disclosure.

FIG. 5 illustrates a schematic representation of the remodeled variational autoencoder network, in accordance with an embodiment of the present disclosure.

FIG 6 is a graphical representation showing comparison of actual chromaticity coordinates and predicted chromaticity coordinates using the remodeled variational autoencoder network, in accordance with an embodiment of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Over the last decade, the data-driven inverse methods for designing nanostructures have gained a significant traction. But as discussed previously, inverse models for plasmonic structures remain a challenge due to the large simulation times required for generating the datasets. Further, the conventional design methods require using first principles in an iterative manner wherein parameters influencing the device response are tweaked till the desired performance is achieved.

Additionally, for plasmonic metasurfaces there can be instances where multiple meta-units may yield near-identical colors implying that tandem networks will be a reasonable choice as they force the model to converge to a single solution. However, this typically requires large, well-distributed datasets. The distribution of the training data pairs plays an important role during inverse design as uneven spreads and clusters hinders learning significantly.

Further, the convolutional neural network based autoencoders are also being used for inverse parameter retrieval. Additionally, the deep learning models, owing to their inherent capability for strong generalization, renders them as an ideal choice for mapping morphological and topological features to electromagnetic responses.

However, the existing techniques have only provided anecdotal evidence for translatability of inverse models into actual fabrication scenarios. In reality, there is a need for design models that are robust to variations within fabrication tolerances. In addition, the colour space prediction suffers from low accuracy in regions corresponding to x and y coordinates proximal to the 45-degree axis in the CIE 1931 colour space.

So, a technique that can inversely and accurately predict the plasmonic metamaterial nanofeatures i.e., the metaunit geometries to achieve structural colour at will while eliminating the need of requiring complex modelling techniques, large datasets and advance computing needs is still to be explored.

Embodiments of the present disclosure overcome the above-mentioned disadvantages, such as large datasets, higher computational expense, low accuracy etc., by providing a system and a method for inversely predicting nanofeatures of a plasmonic metasurface using a remodeled variational autoencoder. More specifically, the system and the method of the present disclosure uses a remodeled variational autoencoder (VAE) network that inversely predicts plasmonic metasurface nanofeatures while providing diversity as well as prediction accuracy. The remodeled VAE network has a probabilistic encoder that maps chromaticity coordinates to a latent space and a probabilistic decoder that maps this latent space back to the coordinates. Further, a multi-layer perceptron (MLP) layer is added to the latent space that considers the reconstruction as well as dimensional prediction, thereby making the training of the network versatile.

In the present disclosure, the system and the method use a Euclidean filter to reduce the size of a dataset, thereby ensuring improved learning of the remodeled VAE network while eliminating the need of large datasets and high computational requirement. Further, the system and the method train the remodeled VAE network on combined loss incorporating reconstruction, dimension, and Kullback-Leibler (KL) divergence loss, thereby ensuring better accuracy throughout the entire colour space (specially near achromatic points).

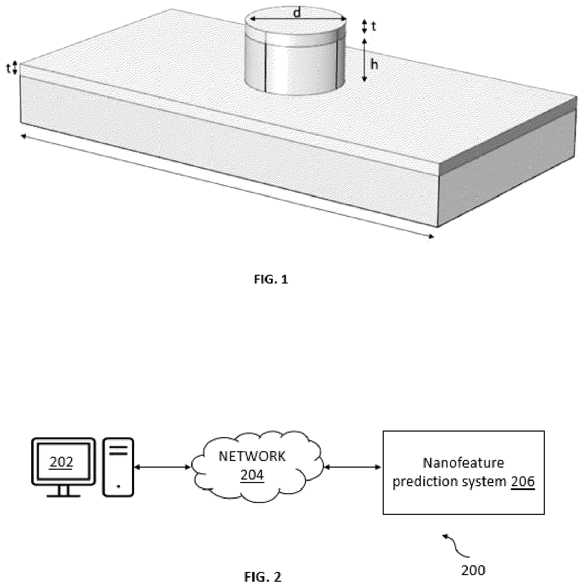

Referring now to the drawings, and more particularly to FIGS. 1 through 6, where similar reference characters denote corresponding features consistently throughout the figures, there are shown preferred embodiments and these embodiments are described in the context of the following exemplary system and/or method.

FIG. 1 illustrates a schematic representation of a polydimethylsiloxane (PDMS) metaunit of a plasmonic metasurface, related to at least some example embodiments of the present disclosure.

As seen in the FIG. 1, 'd', 'h', and 't' represents a diameter, a pillar height, and a thickness of the metaunit, respectively. The 'd', 'h', and '1' can be collectively refereed as 'D' i.e., the metaunit dimension. This 'D', upon coming in contact with incoming radiation, is capable of generating a particular colour 'R' in CIE 1931 colour space.

FIG. 2 illustrates an exemplary representation of an environment 200 related to at least some example embodiments of the present disclosure. Although the environment 200 is presented in one arrangement, other embodiments may include the parts of the environment 200 (or other parts) arranged otherwise depending on, for example, converting spectral response data, creating Euclidean filter, etc. The environment 200 generally includes an electronic device, such as an electronic device 202, and a nanofeature prediction system (hereinafter referred as 'NPS') 206, each coupled to, and in communication with (and/or with access to) a network 204. It should be noted that one electronic device is shown for the sake of explanation; there can be more number of electronic devices.

The network 204 may include, without limitation, a light fidelity (Li-Fi) network, a local area network (LAN), a wide area network (WAN), a metropolitan area network (MAN), a satellite network, the Internet, a fiber optic network, a coaxial cable network, an infrared (IR) network, a radio frequency (RF) network, a virtual network, and/or another suitable public and/or private network capable of supporting communication among two or more of the parts or users illustrated in FIG. 2, or any combination thereof.

Various entities in the environment 200 may connect to the network 204 in accordance with various wired and wireless communication protocols, such as Transmission Control Protocol and Internet Protocol (TCP/IP), User Datagram Protocol (UDP), 2nd Generation (2G), 3rd Generation (3G), 4th Generation (4G), 5th Generation (5G) communication protocols, Long Term Evolution (LTE) communication protocols, or any combination thereof.

The electronic device 202 is associated with a user (e.g., a user or an entity such as an organization) who wants to produce colour at will. Examples of the electronic device 102 include, but are not limited to, a personal computer (PC), a mobile phone, a tablet device, a Personal Digital Assistant (PDA), a server, a voice activated assistant, a smartphone, and a laptop.

The nanofeature prediction system 206 includes one or more hardware processors, such as hardware processors 304 and a memory, such as memory 302. The NPS 206 is configured to perform one or more of the operations described herein. The NPS 206 is configured to receive a spectral response data associated with a plasmonic metasurface (shown in FIG. 1) via the network 204 from the user device 204. The received spectral response data is then filtered using a Euclidian filter to remove clustering of data points in a coordinate space.

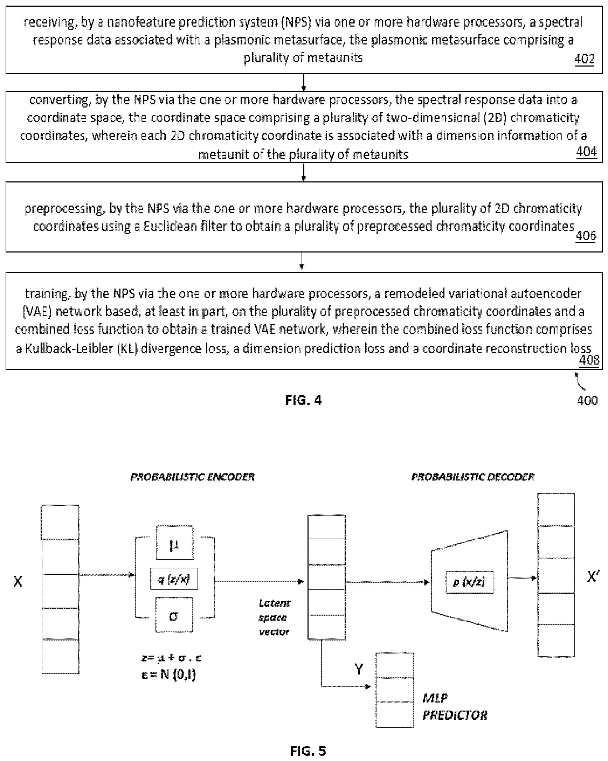

Thereafter, the NPS 206 trains a remodeled variational autoencoder (VAE) network based on the filtered data and a combined loss function to obtain a trained VAE network. The trained VAE network is then used to inversely predict nanofeatures i.e., the dimension information of the metasurface. The process of creating and training the remodeled variational autoencoder network is explained in detail with reference to FIG. 4.

It is assumed that if 'D' denotes the specified metasurface dimensions and 'R' denotes the corresponding colour coordinates in CIE 1931 colour space, then the trained VAE network can predict the required 'D' for user intended 'R' i.e., if a user choose a certain colour in the CIE 1931 colour space, then the trained VAE network can predict the dimension required for the metasurface to obtain that colour.

The number and arrangement of systems, containers, and/or networks shown in FIG. 2 are provided as an example. There may be additional systems, containers, and/or networks; fewer systems, containers, and/or networks; different systems, containers, and/or networks; and/or differently arranged systems, containers, and/or networks than those shown in FIG. 2. Furthermore, two or more systems shown in FIG. 2 may be implemented within a single system or device, or a single system or device shown in FIG. 2 may be implemented as multiple, distributed systems or devices. Additionally, or alternatively, a set of systems (*e.g.,* one or more systems) of the environment 200 may perform one or more functions described as being performed by another set of systems of the environment 200 (e.g., refer scenarios described above).

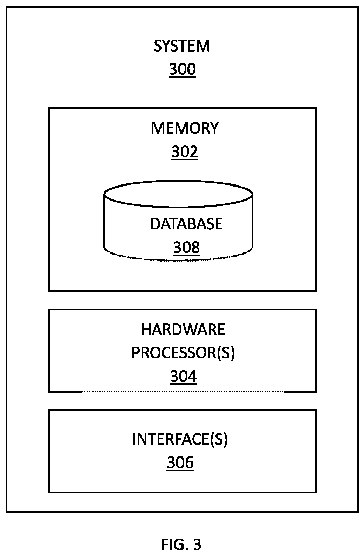

FIG. 3 illustrates an exemplary block diagram of a nanofeature prediction system 300 for inversely predicting nanofeatures of a plasmonic metasurface using the remodeled variational autoencoder, in accordance with an embodiment of the present disclosure. In an embodiment, the nanofeature prediction system 300 may also be referred as a system and may be interchangeably used herein. The system 300 is similar to the NPS 206 explained with reference to FIG. 2. In some embodiments, the system 300 is embodied as a cloud-based and/or SaaS-based (software as a service) architecture. In some embodiments, the system 300 may be implemented in a server system. In some embodiments, the system 300 may be implemented in a variety of computing systems, such as laptop computers, notebooks, hand-held devices, workstations, mainframe computers, and the like.

In an embodiment, the system 300 includes one or more processors 304, communication interface device(s) or input/output (I/O) interface(s) 306, and one or more data storage devices or memory 302 operatively coupled to the one or more processors 304. The one or more processors 304 may be one or more software processing modules and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory.

The I/O interface device(s) 306 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 302 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, a database 308 can be stored in the memory 302, wherein the database 308 may comprise, but are not limited to, a VAE network, loss functions, point selection queue etc. The memory 302 further comprises (or may further comprise) information pertaining to input(s)/output(s) of each step performed by the system and method of the present disclosure. In other words, input(s) fed at each step and output(s) generated at each step are comprised in the memory 302 and can be utilized in further processing and analysis.

FIG. 4, with reference to FIGS. 2 and 3, illustrates an exemplary flow diagram of a method 400 for inversely predicting nanofeatures of the plasmonic metasurface using the remodeled variational autoencoder, in accordance with an embodiment of the present disclosure. The method 400 may use the system 300 of FIG. 3 and NPS 206 of FIG. 2 for execution. In an embodiment, the system 300 comprises one or more data storage devices or the memory 302 operatively coupled to the one or more hardware processors 304 and is configured to store instructions for execution of steps of the method 400 by the one or more hardware processors 304. The sequence of steps of the flow diagram may not be necessarily executed in the same order as they are presented. Further, one or more steps may be grouped together and performed in form of a single step, or one step may have several sub-steps that may be performed in parallel or in sequential manner. The steps of the method 400 of the present disclosure will now be explained with reference to the components of the system 300 as depicted in FIG. 3, and the flow diagram.

At step 402 of the method of the present disclosure, the one or more hardware processors 304 comprised in the system 300 receive a spectral response data associated with the plasmonic metasurface. The plasmonic metasurface includes a plurality of metaunits. The above step 402 is better understood by way of following description.

As discussed previously, 'metasurfaces' are artificially engineered, two-dimensional nanocomposites that possess the ability to demonstrate structural color effects. The metasurfaces include a plurality of periodic sub-wavelength unit cells (also referred as metaunits) that interact with incoming radiation in unconventional to enable manipulation of physical phenomena, such as reflection, absorption, diffraction by varying the metaunit dimensions. So, if the metaunit dimensions are appropriately selected, the selective coloration can be achieved on metasurfaces.

So, the system 300 receives the spectral response data associated with the plasmonic metasurface. The spectral response data is the publicly available data. The spectral response data includes colour coordinates i.e., x and y as per the CIE 1931 colour space and corresponding dimensions i.e., a diameter, a height, and a thickness of the metaunit that can generate that those colours.

At step 404 of the present disclosure, the one or more hardware processors 304 of the system 300 convert the spectral response data into a coordinate space. In particular, the system 300 performs sorting of the spectral response data for visualization purposes. The sorted data is refereed as the coordinate space. The coordinate space includes a plurality of two-dimensional (2D) chromaticity coordinates i.e., x and y coordinates. Further, in the coordinate space, each 2D chromaticity coordinate is associated with a dimension information of a metaunit of the plurality of metaunits. In particular, the coordinate space includes coordinate information of each colour and the dimension information (i.e., the diameter, the height, and the thickness) of each metaunit that can generate the corresponding colour.

At step 406 of the present disclosure, the one or more hardware processors 304 of the system 300 preprocess the plurality of 2D chromaticity coordinates (also referred as 2D chromaticity coordinates and interchangeably used herein) using a Euclidean filter to obtain a plurality of preprocessed chromaticity coordinates. The above step 406 is better understood by way of following description.

The 2D chromaticity coordinates are concentrated within an oblong region around the achromatic (herein after also referred as white point) of the CIE chart. The concentration around the white point results in uneven clustering and thus can cause insufficient learning of a network due to the possibility of obtaining multiple outputs for similar inputs. So, to avoid skewing of the data towards this cluster, the plurality of 2D chromaticity coordinates are filtered using the Euclidean filter to obtain the plurality of preprocessed chromaticity coordinates.

So, for performing filtering, the one or more hardware processors 304 of the system 300 first create the Euclidean filter based on a predefined threshold value. In particular, '5' threshold values were randomly chosen and the reduction in the clustering was evaluated qualitatively for each threshold value. So, the threshold value that provided the best result is selected as the predefined threshold value. It is to be understood by a person having ordinary skill in the art or person skilled in the art that the examples and number of threshold values shall not be construed as limiting the scope of the present disclosure. Thereafter, for each 2D chromaticity coordinate of the plurality of 2D chromaticity coordinate present in the coordinate space, the hardware processors 304 of the system 300 identify a 2D chromaticity coordinate as a first point. Then, the system 300 calculates a Euclidean distance between the first point and a second point present in the coordinate space. It should be noted that the second point is a next 2D chromaticity coordinate available among the plurality of 2D chromaticity coordinates. Further, the system 300 selects the second point among the plurality of 2D chromaticity coordinates based on a comparison of the calculated Euclidean distance and the Euclidean filter. In particular, if the calculated Euclidean distance is found to be greater than the predefined threshold value, the system 300 selects the second point. The second point is then added in a point selection queue based on the selection.

Further, the one or more hardware processors 304 of the system 300 identify the next point available after the second point as the second point in the plurality of 2D chromaticity coordinates and the whole process is repeated until the Euclidean distance between the first point and each 2D chromaticity coordinate of the plurality of 2D chromaticity coordinates is calculated in the coordinate space.

Once the distance calculation process is completed, the one or more hardware processors 304 of the system 300 create the plurality of preprocessed chromaticity coordinates using one or more points available in the point selection queue as the point selection queue includes the required chromaticity coordinates. In one embodiment, the plurality of preprocessed chromaticity coordinates includes one or more 2D chromaticity coordinates, and dimension information of the metaunit corresponding to each 2D chromaticity coordinate of the one or more 2D chromaticity coordinates.

At step 408 of the present disclosure, the one or more hardware processors 304 of the system 300 train a remodeled variational autoencoder (VAE) network based, at least in part, on the plurality of preprocessed chromaticity coordinates and a combined loss function to obtain a trained VAE network. In an embodiment, the combined loss function includes a Kullback-Leibler (KL) divergence loss, a dimension prediction loss and a coordinate reconstruction loss. The above step 408 is better understood by way of following description.

In an embodiment, before training the remodeled VAE network, the one or more hardware processors 304 of the system 300 first develop the remodeled VAE network. So, for developing the remodeled VAE network, the one or more hardware processors 304 of the system 300 access a VAE network. The VAE network is an unsupervised learning method and includes a probabilistic encoder and a probabilistic decoder. Thereafter, the one or more hardware processors 304 of the system 300 remodel the VAE by adding a multi-layer perceptron (MLP) to a latent space of the VAE network to obtain the remodeled VAE network. In an embodiment, the remodeled VAE network includes the probabilistic encoder, the probabilistic decoder, and a MLP predictor.

Once the remodeled VAE network is available, the one or more hardware processors 304 of the system 300 train the remodeled VAE network to obtain the trained VAE network. For training the remodeled VAE network, the one or more hardware processors 304 of the system 300 pass one or more 2D chromaticity coordinates as an input to the probabilistic encoder of the remodeled VAE network. Thereafter, the dimension information of the metaunit corresponding is passed to each 2D chromaticity coordinate of the one or more 2D chromaticity coordinates as an output to the MLP predictor of the remodeled VAE network. The addition of the MLP layer from the latent space to a vector space defined by the dimension information of the metaunit may make the training of the remodeled VAE network versatile by considering the reconstruction as well as dimensional prediction.

Further, the one or more hardware processors 304 of the system 300 train the remodeled VAE network based, at least in part, on the input of the probabilistic encoder, the output to the MLP predictor and the combined loss function to obtain the trained VAE network. The combined loss function has three components i.e., the mean squared error in the reconstruction of the coordinate space, KL divergence loss that captures disparity between the learned distribution and the distribution of the prior network, and the mean squared error in the prediction of dimension through a dense layer added to the latent space. Basically, the remodeled VAE network works on the concept of generative modelling that learns by minimizing loss i.e., the reconstruction, the dimension and KL divergence loss. So, the trained VAE network is capable of predicting the design 'D' parameters from colour coordinates 'R'.

In an embodiment, the VAE network is trained for hundreds of iterations using an Adam optimizer with a learning rate of 0.01.

Once the trained VAE network is obtained, one or more hyperparameters, such as neurons, layer size, activation function, latent space dimension, regularization, optimizer, are tuned to obtain an optimum case. The optimum case is then saved in a database, such as the database 308.

In an embodiment, once the trained VAE network is available, the users can use the system 300 for predicting the required 'D' for particular user intended 'R'. In that case, the one or more hardware processors 304 of the system 300 receives a 2D chromaticity coordinate as an input from a user device, such as the user device 202. Then, the hardware processors 304 of the system 300 obtain a dimension information of a new metaunit corresponding to the new 2D chromaticity coordinate using the trained VAE network that is stored in the database. The dimension information of the new metaunit is then displayed on the user device.

FIG. 5, with reference to FIGS. 1 through 4, illustrates a schematic representation of the remodeled VAE network, in accordance with an embodiment of the present disclosure. As discussed earlier, the remodeled VAE network is trained based on the plurality of preprocessed chromaticity coordinates and a combined loss function to obtain the trained VAE network. The process of training is better understood by way of following description.

As seen in the FIG.5, the remodeled VAE network takes the CIE x and CIE y chromaticity coordinates as inputs for the probabilistic encoder. The output of the MLP yields the metaunit (nanopillar) dimensions. Consider that a distribution $z$ for the latent space is available and the system 300 needs to generate a sample $x$ containing CIE coordinates from this network which is represented by $p(x|z)$. The below mentioned steps needs to be followed.

From Bayes theorem, the following is obtained:

$$p(x|z) = \frac{p(z|x) * p(x)}{p(z)}$$

But it is known that $p(x)$ is difficult to evaluate so this is obtained as:

$$p(x) = \int p(x|z) * p(z)dz$$

So, $p(z|x)$ is approximated as $q(z|x)$ and the difference in the distribution is evaluated using the KL divergence loss. So, the objective becomes a minimization problem mentioned below:

$$\min KL(q(z|x)\|p(z|x)$$

The minimization problem is equivalent to the below mentioned maximization problem:

$$E_{q(z|x)}logp(x|z) - KL\ (q(z|x)\|p(z))$$

Where, $E_{q(z|x)}logp(x|z)$ represents reconstruction likelihood for the coordinate space, and $KL\ (q(z|x)\|p(z))$ represents learned distribution's similarity to the prior distribution.

Then, the combined loss for VAE modifies to:

$$Loss = L(x, \hat{x}) + \sum_{j} KL(q_j(z|x)\|p(z))$$

The MLP layer is now added to the latent space that tries to recreate the dimensions corresponding to the CIE colour coordinate space. So, this loss gets added to the combined loss giving the total loss equation as mentioned below:

$$Loss_{total} = L(y, \hat{y}) + L(x, \hat{x}) + \sum_{j} KL(q_j(z|x)\|p(z)),$$

Where, $L(y, \hat{y})$ represents dimension loss, $L(x, \hat{x})$ represents reconstruction loss and $\Sigma_j KL(q_j(z|x)\|p(z))$ represents KL divergence loss.

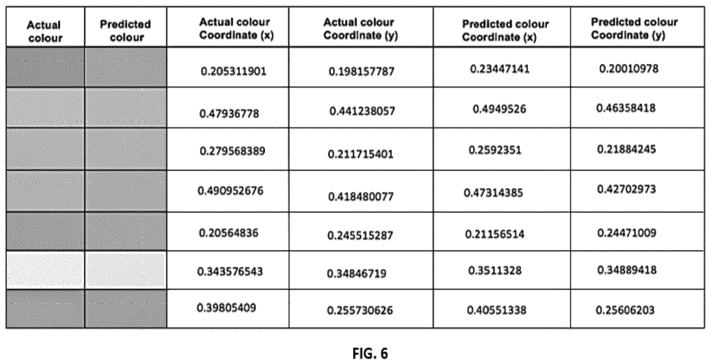

FIG. 6 is a graphical representation showing comparison of actual chromaticity coordinates and predicted chromaticity coordinates using the remodeled VAE network, in accordance with an embodiment of the present disclosure.

As seen in FIG. 6, the predicted chromaticity coordinates are very close to the actual chromaticity coordinates.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

As discussed above, the data-driven inverse methods and tandem autoencoders have been widely used for inversely obtaining plasmonic meta unit dimensions for given structural color(s). However, the disadvantage of the data-driven inverse methods and the tandem autoencoder is that these require a considerably large dataset for inverse prediction, and these also suffer from low accuracy in regions corresponding to x and y coordinates proximal to the 45-degree axis in a CIE 1931 colour space i.e., close to the white point. To overcome the disadvantages, embodiments of the present disclosure provide the method and the system for inversely predicting nanofeatures of the plasmonic metasurface using the remodeled variational autoencoder. More specifically, the system and the method use a Euclidean filter to reduce the size of a dataset, thereby ensuring improved learning of the remodeled VAE network while eliminating the need of large datasets and high computational requirement. Further, the system and the method train the remodeled VAE network on combined loss incorporating reconstruction, dimension, and KL divergence loss, thereby ensuring better accuracy throughout the entire colour space (specially near white points).

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (400), comprising:

   receiving, by a nanofeature prediction system (NPS) via one or more hardware processors, a spectral response data associated with a plasmonic metasurface, the plasmonic metasurface comprising a plurality of metaunits (402);
   converting, by the NPS via the one or more hardware processors, the spectral response data into a coordinate space, the coordinate space comprising a plurality of two-dimensional (2D) chromaticity coordinates, wherein each 2D chromaticity coordinate is associated with a dimension information of a metaunit of the plurality of metaunits (404);
   preprocessing, by the NPS via the one or more hardware processors, the plurality of 2D chromaticity coordinates using a Euclidean filter to obtain a plurality of preprocessed chromaticity coordinates (406); and
   training, by the NPS via the one or more hardware processors, a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss (408).

2. The processor implemented method as claimed in claim 1, wherein the step of preprocessing, by the NPS via the one or more hardware processors, the plurality of 2D chromaticity coordinates using the Euclidean filter to obtain the plurality of preprocessed chromaticity coordinates comprises:

   creating, by the NPS via the one or more hardware processors, the Euclidean filter based on a predefined threshold value;
   for each 2D chromaticity coordinate of the plurality of 2D chromaticity coordinate present in the coordinate space, performing:

      identifying, by the NPS via the one or more hardware processors, a 2D chromaticity coordinate as a first point;
      calculating, by the NPS via the one or more hardware processors, a Euclidean distance between the first point and a second point present in the coordinate space, wherein the second point is a next 2D chromaticity coordinate available among the plurality of 2D chromaticity coordinates;
      selecting, by the NPS via the one or more hardware processors, the second point among the plurality of 2D chromaticity coordinates based on a comparison of the calculated Euclidean distance and the Euclidean filter;
      adding, by the NPS via the one or more hardware processors, the second point in a point selection queue based on the selection; and
      identifying, by the NPS via the one or more hardware processors, a next point available after the second point as the second point in the plurality of 2D chromaticity coordinates,
      until the Euclidean distance between the first point and each 2D chromaticity coordinate of the plurality of 2D chromaticity coordinates is calculated in the coordinate space; and

   creating, by the NPS via the one or more hardware processors, the plurality of preprocessed chromaticity coordinates using one or more points available in the point selection queue, the plurality of preprocessed chromaticity coordinates further comprising one or more 2D chromaticity coordinates, and dimension information of the metaunit corresponding to each 2D chromaticity coordinate of the one or more 2D chromaticity coordinates.

3. The processor implemented method as claimed in claim 2, wherein the step of training, by the NPS via the one or more hardware processors, the remodeled variational autoencoder (VAE) network based, at least in part, on the plurality of preprocessed chromaticity coordinates and the combined loss function to obtain the trained VAE network is preceded by:

accessing, by the NPS via the one or more hardware processors, a VAE network, the VAE network further comprising a probabilistic encoder and a probabilistic decoder; and

remodeling, by the NPS via the one or more hardware processors, the VAE by adding a multi-layer perceptron (MLP) to a latent space of the VAE to obtain the remodeled VAE, the remodeled VAE further comprising the probabilistic encoder, the probabilistic decoder, and a MLP predictor.

4. The processor implemented method as claimed in claim 3, wherein the step of training, by the NPS via the one or more hardware processors, the remodeled VAE network based, at least in part, on the plurality of preprocessed chromaticity coordinates and the combined loss function to obtain the trained VAE network comprises:

passing, by the NPS via the one or more hardware processors, one or more 2D chromaticity coordinates as an input to the probabilistic encoder of the remodeled VAE network;

passing, by the NPS via the one or more hardware processors, the dimension information of the metaunit corresponding to each 2D chromaticity coordinate of the one or more 2D chromaticity coordinates as an output to the MLP predictor of the remodeled VAE network; and

training, by the NPS via the one or more hardware processors, the remodeled VAE network based, at least in part, on the input of the probabilistic encoder, the output to the MLP predictor and the combined loss function to obtain the trained VAE network.

5. The processor implemented method as claimed in claim 1, further comprising:

receiving, by the NPS via the one or more hardware processors, a 2D chromaticity coordinate from a user device;

obtaining, by the NPS via the one or more hardware processors, a dimension information of a new metaunit corresponding to the new 2D chromaticity coordinate using the trained VAE network; and

displaying, by the NPS via the one or more hardware processors, the dimension information of the new metaunit on the user device.

6. A nanofeature prediction system (NPS) (300), comprising:

a memory (302) storing instructions;

one or more communication interfaces (306); and

one or more hardware processors (304) coupled to the memory (302) via the one or more communication interfaces (206), wherein the one or more hardware processors (304) are configured by the instructions to:

receive a spectral response data associated with a plasmonic metasurface, the plasmonic metasurface further comprising a plurality of metaunits;

convert the spectral response data into a coordinate space, the coordinate space further comprising a plurality of two-dimensional (2D) chromaticity coordinates, wherein each 2D chromaticity coordinate is associated with a dimension information of a metaunit of the plurality of metaunits;

preprocess the plurality of 2D chromaticity coordinates using a Euclidean filter to obtain a plurality of preprocessed chromaticity coordinates; and

train a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss.

7. The system as claimed in claim 6, wherein the step of preprocessing the plurality of 2D chromaticity coordinates using the Euclidean filter to obtain the plurality of preprocessed chromaticity coordinates comprises:

create the Euclidean filter based on a predefined threshold value;

for each 2D chromaticity coordinate of the plurality of 2D chromaticity coordinate present in the coordinate space, perform:

identify a 2D chromaticity coordinate as a first point;
calculate a Euclidean distance between the first point and a second point present in the coordinate space, wherein the second point is a next 2D chromaticity coordinate available among the plurality of 2D chromaticity coordinates;
select the second point among the plurality of 2D chromaticity coordinates based on a comparison of the calculated Euclidean distance and the Euclidean filter;
add the second point in a point selection queue based on the selection; and
identify a next point available after the second point as the second point in the plurality of 2D chromaticity coordinates,
until the Euclidean distance between the first point and each 2D chromaticity coordinate of the plurality of 2D chromaticity coordinates is calculated in the coordinate space; and

create the plurality of preprocessed chromaticity coordinates using one or more points available in the point selection queue, the plurality of preprocessed chromaticity coordinates further comprising one or more 2D chromaticity coordinates, and dimension information of the metaunit corresponding to each 2D chromaticity coordinate of the one or more 2D chromaticity coordinates.

8. The system as claimed in claim 7, wherein the step of training the remodeled variational autoencoder (VAE) network based, at least in part, on the plurality of preprocessed chromaticity coordinates and the combined loss function to obtain the trained VAE network is preceded by:

access a VAE network, the VAE network further comprising a probabilistic encoder and a probabilistic decoder; and
remodel the VAE by adding a multi-layer perceptron (MLP) to a latent space of the VAE to obtain the remodeled VAE, the remodeled VAE further comprising the probabilistic encoder, the probabilistic decoder, and a MLP predictor.

9. The system as claimed in claim 8, wherein the step of training the remodeled VAE network based, at least in part, on the plurality of preprocessed chromaticity coordinates and the combined loss function to obtain the trained VAE network comprises:

pass one or more 2D chromaticity coordinates as an input to the probabilistic encoder of the remodeled VAE network;
pass the dimension information of the metaunit corresponding to each 2D chromaticity coordinate of the one or more 2D chromaticity coordinates as an output to the MLP predictor of the remodeled VAE network; and
train the remodeled VAE network based, at least in part, on the input of the probabilistic encoder, the output to the MLP predictor and the combined loss function to obtain the trained VAE network.

10. The system as claimed in claim 6, wherein the one or more hardware processors (304) are configured by the instructions to:

receive a 2D chromaticity coordinate from a user device;
obtain a dimension information of a new metaunit corresponding to the new 2D chromaticity coordinate using the trained VAE network; and
display the dimension information of the new metaunit on the user device.

11. One or more non-transitory machine-readable information storage mediums comprising one or more instructions which when executed by one or more hardware processors cause:

receiving, by a nanofeature prediction system (NPS), a spectral response data associated with a plasmonic metasurface, the plasmonic metasurface further comprising a plurality of metaunits;
converting, by the NPS, the spectral response data into a coordinate space, the coordinate space further comprising a plurality of two-dimensional chromaticity coordinates, wherein each 2D chromaticity coordinate is associated with a dimension information of a metaunit of the plurality of metaunits;
preprocessing, by the NPS, the plurality of 2D chromaticity coordinates using a Euclidean filter to obtain a plurality of preprocessed chromaticity coordinates; and

training, by the NPS, a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss .

12. The one or more non-transitory machine-readable information storage mediums as claimed in claim 11, wherein the step of preprocessing, by the NPS, the plurality of 2D chromaticity coordinates using the Euclidean filter to obtain the plurality of preprocessed chromaticity coordinates comprises:

creating, by the NPS, the Euclidean filter based on a predefined threshold value;
for each 2D chromaticity coordinate of the plurality of 2D chromaticity coordinate present in the coordinate space, performing:

identifying, by the NPS, a 2D chromaticity coordinate as a first point;
calculating, by the NPS, a Euclidean distance between the first point and a second point present in the coordinate space, wherein the second point is a next 2D chromaticity coordinate available among the plurality of 2D chromaticity coordinates;
selecting, by the NPS, the second point among the plurality of 2D chromaticity coordinates based on a comparison of the calculated Euclidean distance and the Euclidean filter;
adding, by the NPS, the second point in a point selection queue based on the selection; and
identifying, by the NPS, a next point available after the second point as the second point in the plurality of 2D chromaticity coordinates,
until the Euclidean distance between the first point and each 2D chromaticity coordinate of the plurality of 2D chromaticity coordinates is calculated in the coordinate space;

creating, by the NPS, the plurality of preprocessed chromaticity coordinates using one or more points available in the point selection queue, the plurality of preprocessed chromaticity coordinates further comprising one or more 2D chromaticity coordinates, and dimension information of the metaunit corresponding to each 2D chromaticity coordinate of the one or more 2D chromaticity coordinates.

13. The one or more non-transitory machine-readable information storage mediums as claimed in claim 12, wherein the step of training, by the NPS, the remodeled variational autoencoder (VAE) network based, at least in part, on the plurality of preprocessed chromaticity coordinates and the combined loss function to obtain the trained VAE network is preceded by:

accessing, by the NPS, a VAE network, the VAE network further comprising a probabilistic encoder and a probabilistic decoder; and
remodeling, by the NPS, the VAE by adding a multi-layer perceptron (MLP) to a latent space of the VAE to obtain the remodeled VAE, the remodeled VAE further comprising the probabilistic encoder, the probabilistic decoder, and a MLP predictor.

14. The one or more non-transitory machine-readable information storage mediums as claimed in claim 13, wherein the step of training, by the NPS, the remodeled VAE network based, at least in part, on the plurality of preprocessed chromaticity coordinates and the combined loss function to obtain the trained VAE network further comprises:

passing, by the NPS, one or more 2D chromaticity coordinates as an input to the probabilistic encoder of the remodeled VAE network;
passing, by the NPS, the dimension information of the metaunit corresponding to each 2D chromaticity coordinate of the one or more 2D chromaticity coordinates as an output to the MLP predictor of the remodeled VAE network; and
training, by the NPS, the remodeled VAE network based, at least in part, on the input of the probabilistic encoder, the output to the MLP predictor and the combined loss function to obtain the trained VAE network.

15. The one or more non-transitory machine-readable information storage mediums as claimed in claim 11, wherein the one or more instructions which when executed by the one or more hardware processors further cause:

receiving, by the NPS, a 2D chromaticity coordinate from a user device;
obtaining, by the NPS, a dimension information of a new metaunit corresponding to the new 2D chromaticity coordinate using the trained VAE network; and

displaying, by the NPS, the dimension information of the new metaunit on the user device.

FIG. 1

FIG. 2

SYSTEM
300

MEMORY
302

DATABASE
308

HARDWARE
PROCESSOR(S)
304

INTERFACE(S)
306

FIG. 3

receiving, by a nanofeature prediction system (NPS) via one or more hardware processors, a spectral response data associated with a plasmonic metasurface, the plasmonic metasurface comprising a plurality of metaunits                                                                                                  402

converting, by the NPS via the one or more hardware processors, the spectral response data into a coordinate space, the coordinate space comprising a plurality of two-dimensional (2D) chromaticity coordinates, wherein each 2D chromaticity coordinate is associated with a dimension information of a metaunit of the plurality of metaunits                                                                                    404

preprocessing, by the NPS via the one or more hardware processors, the plurality of 2D chromaticity coordinates using a Euclidean filter to obtain a plurality of preprocessed chromaticity coordinates      406

training, by the NPS via the one or more hardware processors, a remodeled variational autoencoder (VAE) network based, at least in part, on the plurality of preprocessed chromaticity coordinates and a combined loss function to obtain a trained VAE network, wherein the combined loss function comprises a Kullback-Leibler (KL) divergence loss, a dimension prediction loss and a coordinate reconstruction loss      408

**FIG. 4**                                                                                                    400

PROBABILISTIC ENCODER                                    PROBABILISTIC DECODER

X

$\mu$

$q(z/x)$

$\sigma$

$z = \mu + \sigma . \epsilon$
$\epsilon = N(0,I)$

Latent space vector

$p(x/z)$

X'

Y

MLP PREDICTOR

**FIG. 5**

| Actual colour | Predicted colour | Actual colour Coordinate (x) | Actual colour Coordinate (y) | Predicted colour Coordinate (x) | Predicted colour Coordinate (y) |
|---|---|---|---|---|---|
|  |  | 0.205311901 | 0.198157787 | 0.23447141 | 0.20010978 |
|  |  | 0.47936778 | 0.441238057 | 0.4949526 | 0.46358418 |
|  |  | 0.279568389 | 0.211715401 | 0.2592351 | 0.21884245 |
|  |  | 0.490952676 | 0.418480077 | 0.47314385 | 0.42702973 |
|  |  | 0.20564836 | 0.245515287 | 0.21156514 | 0.24471009 |
|  |  | 0.343576543 | 0.34846719 | 0.3511328 | 0.34889418 |
|  |  | 0.39805409 | 0.255730626 | 0.40551338 | 0.25606203 |

FIG. 6

| | Europäisches Patentamt European Patent Office Office européen des brevets | **EUROPEAN SEARCH REPORT** | **Application Number** EP 23 21 9067 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROBERTS NATHAN BRYN ET AL: "A deep learning approach to the forward prediction and inverse design of plasmonic metasurface structural color", APPLIED PHYSICS LETTERS, AMERICAN INSTITUTE OF PHYSICS, 2 HUNTINGTON QUADRANGLE, MELVILLE, NY 11747, vol. 119, no. 6, 9 August 2021 (2021-08-09), XP012258770, ISSN: 0003-6951, DOI: 10.1063/5.0055733 [retrieved on 2021-08-09] * abstract; figures 1,3,5 * | 1-15 | INV. G16C60/00 |
| X | LIU ZHAOCHENG ET AL: "A Hybrid Strategy for the Discovery and Design of Photonic Structures", IEEE JOURNAL ON EMERGING AND SELECTED TOPICS IN CIRCUITS AND SYSTEMS, IEEE, PISCATAWAY, NJ, USA, vol. 10, no. 1, 27 January 2020 (2020-01-27), pages 126-135, XP011777483, ISSN: 2156-3357, DOI: 10.1109/JETCAS.2020.2970080 [retrieved on 2020-03-10] * abstract * * page 129, column 1, paragraph 3; figure 4 * | 1-15 | |

TECHNICAL FIELDS SEARCHED (IPC)

G16C
G06N

-/--

The present search report has been drawn up for all claims

| Place of search | Date of completion of the search | Examiner |
|---|---|---|
| The Hague | 27 May 2024 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 21 9067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | PRAJITH PILLAI: "Modified variational autoencoder for inversely predicting plasmonic nanofeatures for generating structural color", SCIENTIFIC REPORTS, vol. 13, no. 1, 2 March 2023 (2023-03-02), XP093164701, US ISSN: 2045-2322, DOI: 10.1038/s41598-023-30069-1 Retrieved from the Internet: URL:https://www.nature.com/articles/s41598-023-30069-1> * the whole document * | 1-15 | |

TECHNICAL FIELDS
SEARCHED     (IPC)

The present search report has been drawn up for all claims

| Place of search | Date of completion of the search | Examiner |
|---|---|---|
| The Hague | 27 May 2024 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221074995